# EUROPÄISCHE PATENTANMELDUNG

(11) **EP 4 631 379 A1**
(43) Veröffentlichungstag der Anmeldung: **15.10.2025**
(21) Anmeldenummer: 24200754.0
(22) Anmeldetag: 17.09.2024
(51) Int. Cl.: A41D 13/00, A41D 13/12, A41D 27/20

(54) **KLEIDUNGSSTÜCK ZUR AUFNAHME EINER MEDIZINISCHEN PUMPE**

(30) Priorität: 11.04.2024 AT 503082024
(71) Anmelder: Ouele, Kounoungdiga Elisabeth, 6345 Kössen (AT)
(72) Erfinder: Ouele, Kounoungdiga Elisabeth, 6345 Kössen (AT)
(74) Vertreter: Schwarz & Partner Patentanwälte GmbH

(57) **Zusammenfassung**

Kleidungsstück (1) zur Aufnahme und zum Transport einer medizinischen Pumpe, umfassend: (a) ein textiles Oberteil (2), (b) eine textile Hose (3) mit einem Hosenbund (4) und (c) zumindest eine Tasche (6, 6') zur Aufnahme und zum Transport der medizinischen Pumpe; wobei die Tasche (6, 6`) an der Innenseite der textilen Hose (3) angeordnet ist, wobei die Tasche (6, 6') nach oben hin eine Öffnung (7, 7') zum Einstecken der medizinischen Pumpe aufweist, wobei die Öffnung (7, 7') unterhalb des Hosenbunds (4) angeordnet, wobei die textile Hose (3) am Hosenbund (4) mit dem textilen Oberteil (2) über eine lösbare Verbindung 5 lösbar verbindbar ist.

## Beschreibung

Die vorliegende Erfindung betrifft ein Kleidungsstück zur Aufnahme und zum Transport einer medizinischen Pumpe, umfassend ein textiles Oberteil, eine textile Hose mit einem Hosenbund und zumindest eine Tasche zur Aufnahme und zum Transport der medizinischen Pumpe.

### HINTERGRUND DER ERFINDUNG

Personen, die für eine Dauermedikation ständig medizinische Pumpen bei sich tragen müssen, stehen oft vor der Herausforderung diese Geräte sicher und bequem bei sich zu führen, ohne dass ihre täglichen Aktivitäten beeinträchtigt werden. Außerdem gibt es Erkrankungen, bei denen eine Person krankheitsbedingt unkontrollierte Bewegungen macht, die dazu führen können, dass die Versorgung der Person mit einem Arzneimittel aus der Pumpe beeinträchtigt wird.

Bei Parkinsonpatienten kommen im fortgeschrittenen Stadium der Krankheit derartige Pumpensysteme zur Verabreichung von Medikamenten zum Einsatz, wenn die oral verabreichten Medikamente nicht mehr ausreichen und die Symptome effektiver kontrolliert werden sollen oder wenn die Patienten erhebliche Schwankungen in ihrer Symptomkontrolle erfahren. Die Pumpensysteme liefern Medikamente direkt und kontinuierlich, um eine stabilere Kontrolle der Symptome zu ermöglichen. Bei der Levodopa/Carbidopa-Intestinalgel-Pumpe liefert eine gelöste Form von Levodopa/Carbidopa die Arzneimittel über eine kleine, durch die Bauchwand eingeführte Sonde direkt in den Dünndarm. Dieses System umgeht den Magen, wodurch die Aufnahme des Medikaments weniger von der Magenentleerung beeinflusst wird, und eine gleichmäßigere und kontinuierlichere Medikamentenversorgung ermöglicht wird. Bei der Apomorphin-Pumpe liefert das Pumpensystem kontinuierlich kleine Dosen Apomorphin über einen längeren Zeitraum, was eine gleichmäßigere Symptomkontrolle ermöglicht und die "Off"-Phasen reduziert, in denen die Wirkung der Parkinson-Medikamente nachlässt und die Symptome wieder stärker werden.

Diese Pumpensysteme erfordern eine sorgfältige Handhabung und Überwachung durch geschultes medizinisches Personal und die Patienten selbst. Herkömmliche Kleidungsstücke bieten oft nicht die notwendige Unterstützung oder Sicherheit, was zu Unannehmlichkeiten oder sogar zu Schäden am Gerät führen kann.

US 5,048,122 zeigt einen Body, der innen im Brustbereich eine Aufsatztasche mit Klettverschluss aufweist, die zum Verstauen einer Katheter-Leitung dient. Die Tasche ist aus zwei Stoffschichten gebildet. Der Body hat auf der Rückseite einen Reißverschluss, der so ausgeführt ist, dass er nicht auf der Haut anliegt.

US 2015/216242 offenbart ein Oberteil, das innen im Hüftbereich Taschen zur Aufnahme von Drainagebeutel aufweist. Die Taschen sind aus wasserfestem, leicht zu reinigendem Material hergestellt.

US 2016/050995 beschreibt ein Oberteil mit einer Aufsatztasche für medizinische Geräte. Die Tasche weist eine Öffnung nach Innen auf und ist mit Halterungen für die medizinischen Geräte ausgestattet.

Der Figur 2 von DE 196 03 232 ist ein Body zu entnehmen, der im unteren Bereich wie ein Slip hergestellt ist und im Bereich des Bauchnabels mit dem Oberteil über Häkchen, Druckknöpfe oder einen Reißverschluss verbunden wird. Der Verschluss ist weich unterlegt, so dass er nicht an der Haut anliegt.

In DE 20 2005 005 692 U2 ist eine Spielerbekleidung dargestellt, die aus einer Hose und einem Oberteil besteht. Die beiden Teile sind durch einen vollständig um die Hüfte verlaufenden Reißverschluss miteinander verbunden.

### KURZBESCHREIBUNG DER ERFINDUNG

Im Stand der Technik sind Kleidungsstücke bekannt, die eine Aufnahme einer Pumpe ermöglichen. Allerdings sind diese Kleidungsstücke entweder kompliziert in der Handhabung oder sie gewährleisten nicht, dass die Pumpe sicher fixiert wird oder dass ein von der Pumpe wegführender Schlauch nicht abgeklemmt oder beschädigt wird.

Aufgabe der vorliegenden Erfindung ist daher die Bereitstellung eines Kleidungsstücks zur Aufnahme und zum Transport einer medizinischen Pumpe, welches einen sicheren Transport bzw. eine sichere Verwahrung der Pumpe am Körper ermöglicht und dennoch einfach in der Handhabung ist.

Gelöst wird diese Aufgabe durch ein Kleidungsstück zur Aufnahme und zum Transport einer medizinischen Pumpe, umfassend:
- ein textiles Oberteil,
- eine textile Hose mit einem Hosenbund,
- zumindest eine Tasche zur Aufnahme und zum Transport der medizinischen Pumpe, dadurch gekennzeichnet, dass
   die Tasche an der Innenseite der Hose angeordnet ist,
   wobei die Tasche nach oben hin eine Öffnung zum Einstecken der medizinischen Pumpe aufweist, wobei die Öffnung unterhalb des Hosenbunds angeordnet,
   wobei die textile Hose am Hosenbund mit dem textilen Oberteil lösbar verbindbar ist.

Das textile Oberteil kann z.B. ein T-Shirt sein. Die textile Hose kann z.B. nach Art einer Shorts oder als Slip ausgebildet sein und sie weist einen Hosenbund auf, d.h. einen Saumabschluss nach oben. Die Tasche ist an der Innenseite der Hose, d.h. im getragenen Zustand an der dem Körper zugewandten Seite, angeordnet. Diese ist derart ausgebildet, dass sie sich zur Aufnahme und zum Transport der medizinischen Pumpe eignet. D.h. die Tasche weist eine entsprechende Dimension auf, dass eine medizinische Pumpe eingesetzt und verwahrt werden kann. Diese Tasche ist nach oben hin mit einer Öffnung versehen, die eine Größe aufweist, dass sie ein Einstecken der medizinischen Pumpe ermöglicht. Nach oben hin offen bezieht sich - wie alle Positionsangaben bezogen auf das Kleidungsstück - auf das von einer aufrechtstehenden Person getragene Kleidungsstück. Die Öffnung bzw. die Oberkannte der Öffnung ist unterhalb des Hosenbunds angeordnet. Die textile Hose ist am Hosenbund mit dem textilen Oberteil lösbar verbindbar. Die Anordnung der Öffnung zum Körper hin führt dazu, dass diese von außen nicht ohne Weiteres zugänglich ist. Mit dem Einführen der Pumpe in die Tasche verdeckt also bereits die Hose selbst im getragenen Zustand den Zugang von außerhalb der Hose. Die Öffnung befindet sich also zwischen Körper und Außenseite der Hose. Ein Zugang zur Öffnung - und in weiterer Folge zur Hose - kann nur erfolgen, indem man zwischen Hose und Körper eingreift.

Durch die Verbindung der Hose am Bund mit dem Oberteil wird darüber hinaus der unmittelbare Zugang zur Öffnung unmöglich gemacht, da das mit der Hose verbundene Oberteil den Weg zwischen Körper und Hoseninnenseite versperrt. Außerdem wird durch die Verbindung von Oberteil und Hose ein zusammengesetztes Kleidungsstück wie ein Body gebildet. Ein Body oder Bodysuit (engl. "Körperanzug") ist ein meist enganliegendes, den Rumpf bedeckendes Kleidungsstück und komfortabel zu tragen. Die vorliegende Erfindung betrifft also ein Kleidungsstück, das dafür speziell entworfen wurde, eine medizinische Pumpe sicher und komfortabel zu tragen.

Das Kleidungsstück kann atmungsaktiv sein, um für einen langen Tragekomfort zu sorgen. Als Materialien kommen für das Kleidungsstück in der Textilindustrie bekannte Materialien bestehen, z.B. aus einem flexiblen, hautfreundlichen und/oder dehnbarem Material.

Weiters kann vorgesehen sein, dass die Öffnung einen Verschluss zum Verschließen der Öffnung aufweist. Der Verschluss kann z.B. ein Klettverschluss oder ein Reißverschluss sein. Auf diese Weise ist gesichert, dass auch in einer Zeitspanne, in welcher das Oberteil von der Hose gelöst ist, kein unmittelbarer Zugang zur Pumpe möglich ist. Ein Lösen der Verbindung ist z.B. für Zeiten der Körperhygiene unerlässlich. Zusätzlich wird die Pumpe in der Tasche auch gegen Beschädigung durch Feuchte von außen besser abgesichert.

Die Anordnung der Tasche an der Innenseite hat neben den bereits genannten Vorteilen auch noch den Zusatznutzen, dass der von der Pumpe zum Körper führende Schlauch vom Kleidungsstück im getragenen Zustand vollständig überdeckt ist, d.h. nie an die Oberfläche des Kleidungsstückes gelangen muss. Für die ideale Positionierung des Schlauchs am Körper kann darüber hinaus die Tasche einen Auslass aufweisen, durch den der Schlauch der Pumpe ausführbar ist, wobei der Auslass zur Innenseite der Hose gerichtet ist. Derart kann die Verlegung des Schlauchs am Körper besonders gezielt erfolgen und es ist eine sichere Ausführung des Schlauchs von der Pumpe zum Ort der Zufuhr des Arzneimittels in den Körper der Person möglich.

Das Oberteil und die Hose können mittels bekannter Verbindungsmittel miteinander verbunden werden. Die sicherste und gleichzeitig komfortabelste Verbindung erfolgt mittels eines Reißverschlusses. Auf diese Art lassen sich Hose und Oberteil leicht lösen und verbinden und der Zugang bzw. die Vermeidung des Zugangs zur Öffnung der Tasche lässt sich je einfach und sicher bewerkstelligen.

Bevorzugt ist der Reißverschluss so ausgebildet, dass er zwei Seitenteile und einen Schieber umfasst, wobei die Seitenteile jeweils Krampen aufweisen, wobei die Krampen der Seitenteile mittels des Schiebers miteinander formschlüssig verbindbar sind, wobei ein Seitenteil am Bund der textilen Hose und ein Seitenteil an der Innenseite des Oberteils angeordnet ist. Der Schieber ist in der Regel an einem der Seitenteile beweglich eingesetzt. Durch Positionierung der Seitenteile aneinander kann mit dem Schieber die Verbindung von Hose und Oberteil hergestellt werden, indem die Krampen der Seitenteile ineinander eingreifen. Die Seitenteile können dabei sowohl am Oberteil als auch an der Hose derart befestigt sein, dass die Krampen nicht in Kontakt mit der Haut gelangen. Dazu sind die Krampen der Hose an der Außenseite des Bunds angeordnet. Diese Anordnung des Reißverschlusses erhöht den Tragekomfort weiter.

Bei einer Verbindung mittels eines Reißverschlusses kann sich das Seitenteil des Oberteils um zumindest ¾ des Umfangs, vorzugweise des gesamten Umfangs des Oberteils und das Seitenteil des Unterteils passend zum Seitenteil des Oberteils um zumindest ¾ des Umfangs, vorzugweise des gesamten Umfangs der Hose erstrecken. Wenn der Reißverschluss um den größten Teil des Umfangs erstreckt, ist die Sicherung der Pumpe vor Zugriff größer und der Charakter des Kleidungsstücks entspricht dem eines tragekomfortablen Bodys.

Das Oberteil kann an der Außenseite eine weitere Tasche zur Aufnahme und zum Transport einer medizinischen Pumpe aufweisen, wobei die weitere Tasche eine Öffnung aufweist, welche von der Seite zugänglich ist und eine Größe aufweist, dass die Pumpe in die Öffnung steckbar ist, wobei die Öffnung der weiteren Tasche verschließbar ist, vorzugsweise mittels eine Reißverschlusses. Dies ist vorteilhaft für Situationen, in denen der Patient z.B. die Hose ausziehen und das Oberteil weiterhin getragen werden soll. Die Pumpe kann dann vorübergehend in die weitere Tasche gesteckt werden.

Zusätzlich oder alternativ dazu kann eine weitere Tasche als Brusttasche vorgesehen sein. Diese kann ebenfalls einen Verschluss, z.B. einen Klettverschluss aufweisen.

Sämtliche oder nur einzelne Taschen, die zur Aufnahme und zum Transport der medizinischen Pumpe vorgesehen sind, können integrierte Verstärkungsbänder aufweisen. Die Verstärkungsbänder sind bevorzugt aus Kunststoff gefertigt. Die Verstärkungsbänder haben die Aufgabe, das Textil im Bereich der Tasche von der Masse der Pumpe zu entlasten und ein Ausleiern der Tasche zu verhindern, wodurch die Sicherheit und der Komfort des Trägers erhöht werden. Daher sollen die Verstärkungsbänder so positioniert und dimensioniert sein, dass sie die Tasche effektiv unterstützen und die Pumpe stützten. Dazu können die Verstärkungsbänder quer zur Einsteckrichtung positioniert sein und die Tasche vollständig oder zumindest zur Hälfte des Querschnittumfangs der Tasche umgeben. Die Verstärkungsbänder können in das Material der Tasche integriert sein und die seitliche Tasche sowie die gegebenenfalls weitere Tasche verstärken.

Durch die Integration von Taschen, die mit Verstärkungsbändern aus Kunststoff verstärkt sind, bietet das Kleidungsstück eine langlebige und praktische Lösung für Nutzer, die medizinische Pumpen wie Insulinpumpen, Schmerzpumpen oder ähnliche Geräte im Alltag tragen müssen.

Weiter kann vorgesehen sein, dass sämtliche oder nur einzelne Taschen, die zur Aufnahme und zum Transport der medizinischen Pumpe vorgesehen sind, wasserfest ausgebildet sind. Dies erfüllt zwei Funktionen: Einerseits wird im Falle einer Beschädigung der medizinischen Pumpe, z.B. im Bereich der Verbindungsstelle von Pumpe und Schlauch, ein Auslaufen von Flüssigkeit aus der Tasche verhindert und der Patient wird nicht nass. Andererseits stellt die Wasserfestigkeit einen zusätzlichen Schutz der medizinischen Pumpe dar, was im Falle eines unbeabsichtigten Kontakts des Kleidungsstücks mit Wasser von außerhalb eine Beschädigung der Pumpe verhindert.

Die Wasserfestigkeit kann z.B. durch eine wasserdichte Beschichtung erzielt werden. Dazu kann die Innenseite der Tasche eine wasserdichte Beschichtung, bspw. eine Gummibeschichtung, aufweisen.

Die Erfindung kann weitere Merkmale umfassen, wie verstellbare Taschengrößen für verschiedene Pumpenmodelle. Die Hose kann z.B. einen Hosenträger umfassen, mit welchem die Hose an den Schultern eines Patienten befestigbar ist, um die Hose besser zu fixieren. Auch kann die Hose nach unten hin öffenbar sein, um ein leichteres An- und Ausziehen zu ermöglichen.

### DETAILLIERTE BESCHREIBUNG DER ERFINDUNG

Weitere Details der Erfindung werden nachfolgend anhand der beiliegenden Figuren erläutert.
- Fig. 1a, 1b: zeigen die Vorderseite eines Oberteils eines erfindungsgemäßen Kleidungsstücks, wobei Fig. 1a die Außenseite des Oberteils zeigt und Fig. 1b die Innenseite des Oberteils zeigt.
- Fig. 2a, 2b: zeigen die Vorderseite einer Hose eines erfindungsgemäßen Kleidungsstücks, wobei Fig. 1a die Außenseite der Hose zeigt und Fig. 1b die Innenseite der Hose zeigt.
- Fig. 3: zeigt die Vorderseite eines Kleidungsstücks gemäß der Erfindung, bei dem das Oberteil aus Fig. 1a mit dem Unterteil aus Fig. 2a verbunden ist.

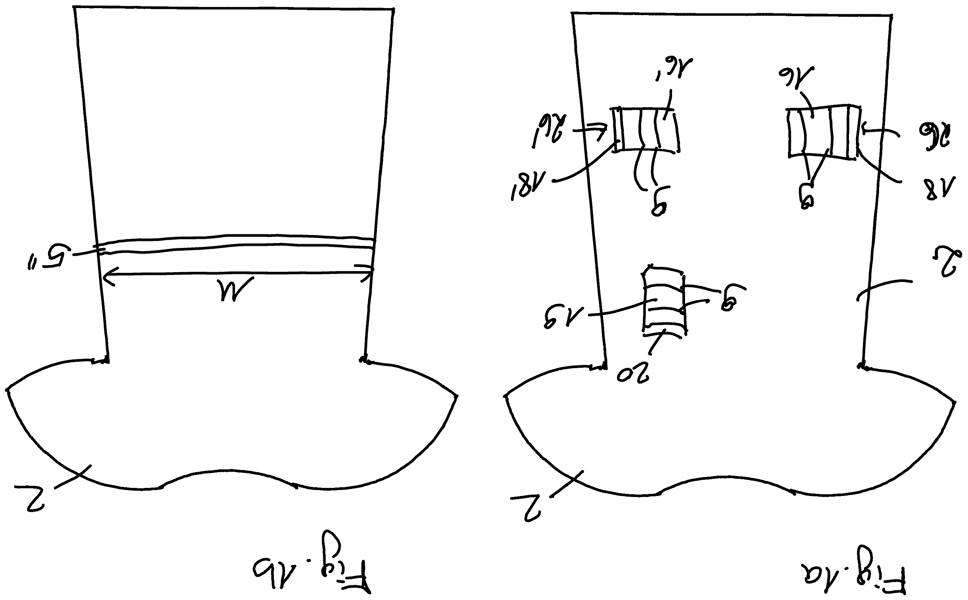

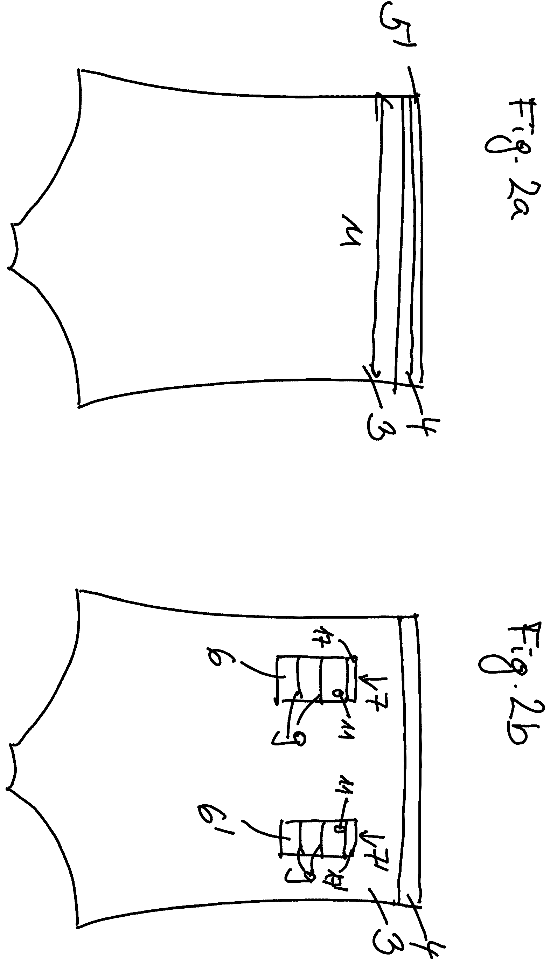

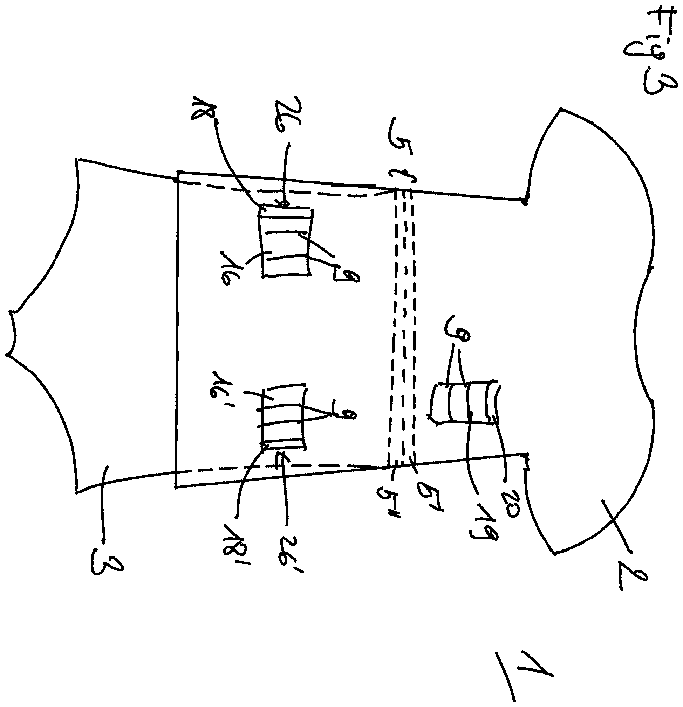

Die Fig. 1a bis 3 zeigen das Oberteil 2 (Fig. 1a, 1b), die Hose 3 (Fig. 2a, 2b) und das daraus gebildete gesamte Kleidungsstück 1 (Fig. 3) zum Verbinden des Oberteils 3 mit der Hose 3 einer Ausführungsvariante der Erfindung. Das Kleidungsstück 1 ist zur Aufnahme und zum Transport einer medizinischen Pumpe ausgebildet und es umfasst ein textiles Oberteil 2 und eine textile Hose 3, die gemeinsam für die Fig. 1a bis 3 gemeinsam beschrieben werden.

Das Oberteil 2 ist im gezeigten Ausführungsbeispiel als T-Shirt ausgebildet und enganliegend, den Rumpf einer Person bedeckend ausgebildet.

Die textile Hose 3 ist als enganliegender Slip ausgebildet und sie weist einen Hosenbund 4 auf, wobei sich ein Seitenteil 5' eines Reißverschlusses 5 als Verbindungsmittel zwischen Hose 3 und Oberteil 2 an der Außenseite der Hose 3 - etwa am oberen Ende des Hosenbunds 4 - befindet.

Das zweite Seitenteil 5" des Reißverschlusses 5 befindet sich am Oberteil 2 und zwar an der Innenseite etwa auf der Bauchhöhe. Jedes der beiden Seitenteile (5', 5") weist Krampen auf, wobei die Krampen der Seitenteile 5', 5" mittels eines nicht gezeigten Schiebers miteinander formschlüssig verbindbar sind. Im verbundenen Zustand überdeckt der untere Teil 2' des Oberteils 2 die Hose 3.

Die Hose 3 weist zwei Taschen 6, 6' zur Aufnahme und zum Transport einer medizinischen Pumpe auf. Die Taschen 6, 6' sind an der Innenseite der textilen Hose 3 angeordnet, wobei beide Taschen 6, 6' nach oben hin jeweils eine Öffnung 7, 7' mit einer Größe aufweisen, dass eine medizinische Pumpe eingesteckt werden kann. Die Öffnungen 7, 7' sind unterhalb des Hosenbunds 4 angeordnet.

Jeder der Öffnungen 7, 7' ist ein Verschluss 17, 17' zum Verschließen der Öffnung 7, 7' zugeordnet. Der Verschluss 17, 17' ist als Klettverschluss ausgebildet. Zum Ausführen eines Schlauchs der Pumpe aus der Tasche 6, 6' heraus ist ein Auslass 11, 11' vorgesehen, durch den der Schlauch der Pumpe geschoben werden kann. Der Auslass 11, 11' ist zur Innenseite der Hose 3 gerichtet, d.h. im getragenen Zustand zum Körper der tragenden Person hin. Im gezeigten Beispiel sind die Auslässe eher am oberen Ende der Tasche, zur Mitte hin orientiert. Genauso gut können die Auslässe am unteren Ende der Tasche und / oder mittig oder nach außen orientiert platziert sein.

Das Seitenteil 5" des Oberteils 2 erstreckt sich den gesamten Umfang U' des Oberteils 2 und das Seitenteil 5 des Unterteils 3 erstreckt sich - passend zum Seitenteil 5" des Oberteils 2 - um den gesamten Umfang U der Hose 3. Wenn der Reißverschluss über jeweils den gesamten Umfang U, U' von Oberteil 2 und Hose 3 verschlossen wird, ist die Pumpe vor Zugriffen vollständig geschützt. Das Kleidungsstück 1 ist dann außerdem ein Body bzw. Bodysuit.

Weiters kann das Oberteil 2 an der Außenseite weitere Taschen 16, 16' zur Aufnahme und zum Transport einer medizinischen Pumpe aufweisen. Die weiteren Taschen 16, 16' weisen jeweils eine Öffnung 18, 18' auf, welche von der Seite des Kleidungsstücks zugänglich sind und eine Größe aufweisen, dass die Pumpe in die Öffnung 18, 18' gesteckt werden können. Die Öffnungen 18, 18' der zusätzlichen Taschen 16, 16' können mit einem Verschlusses 26; 26' z.B. in Form eines Klettverschlusses verschlossen werden. Auch eine Brusttasche 19 kann vorgesehen sein, die z.B. einen Verschluss 20 aufweist.

Sämtliche Taschen 6, 6`; 16, 16`; 19 weisen zur Aufnahme und zum Transport der medizinischen Pumpe integrierte Verstärkungsbänder 9 auf. Diese sind quer zur Einsteckrichtung der Pumpe orientiert. Die Verstärkungsbänder 9 sind aus Kunststoff gefertigt und in den Stoff des Textils integriert. Weiters erstrecken sich die Verstärkungsbänder 9 über den halben Umfang der Taschen 6, 6`; 16, 16`; 19

Die Taschen 6, 6`; 16, 16`; 19 weisen im gezeigten Ausführungsbeispiel eine wasserdichte Beschichtung auf, um ein Auslaufen von Flüssigkeit aus der Pumpe oder ein Nasswerden der Pumpe von außerhalb zu verhindern. Die Taschen 6, 6`; 16, 16`; 19 können außerdem mit einer Polsterung ausgestattet sein (nicht gezeigt), welche sich im getragenen Zustand des Kleidungsstücks 1 zwischen Körper und medizinischer Pumpe befindet. Dadurch wird der Tragekomfort erhöht, da die Pumpe zum Körper auf Distanz gehalten wird.

Das Kleidungsstück 1 kann atmungsaktiv sein, um für einen langen Tragekomfort zu sorgen. Weiters kann das Kleidungsstück 1 kann aus einem flexiblen, hautfreundlichen und dehnbaren Material bestehen.

## Patentansprüche

1. Kleidungsstück (1) zur Aufnahme und zum Transport einer medizinischen Pumpe, umfassend:
• ein textiles Oberteil (2),
• eine textile Hose (3) mit einem Hosenbund (4),
• zumindest eine Tasche (6, 6') zur Aufnahme und zum Transport der medizinischen Pumpe,
**dadurch gekennzeichnet, dass**
die Tasche (6, 6') an der Innenseite der textilen Hose (3) angeordnet ist,
wobei die Tasche (6, 6') nach oben hin eine Öffnung (7, 7') zum Einstecken der medizinischen Pumpe aufweist, wobei die Öffnung (7, 7') unterhalb des Hosenbunds (4) angeordnet,
wobei die textile Hose (3) am Hosenbund (4) mit dem textilen Oberteil (2) über eine lösbare Verbindung 5 lösbar verbindbar ist.

2. Kleidungsstück nach Anspruch 1, **dadurch gekennzeichnet, dass** die Öffnung (7, 7') einen Verschluss (17, 17') zum Verschließen der Öffnung (7, 7') aufweist.

3. Kleidungsstück nach Anspruch 1 oder Anspruch 2, **dadurch gekennzeichnet, dass** die Tasche (6, 6') einen Auslass (11, 11') aufweist, durch den ein Schlauch der Pumpe ausführbar ist, wobei der Auslass (11, 11') zur Innenseite der Hose (3) gerichtet ist.

4. Kleidungsstück nach einem der Ansprüche 1 bis 3, **dadurch gekennzeichnet, dass** die lösbare Verbindung (5) zwischen Oberteil (2) und die Hose (3) einen Reißverschluss umfasst.

5. Kleidungsstück nach Anspruch 4, **dadurch gekennzeichnet, dass** der Reißverschluss zwei Seitenteile (5', 5") und einen Schieber umfasst, wobei die Seitenteile (5', 5") jeweils Krampen aufweisen, wobei die Krampen der Seitenteile (5', 5") mittels des Schiebers miteinander formschlüssig verbindbar sind, wobei ein Seitenteil (5') am Bund (4) der textilen Hose und ein Seitenteil (5") an der Innenseite des Oberteils (2) angeordnet ist.

6. Kleidungsstück nach Anspruch 5, **dadurch gekennzeichnet, dass** sich das Seitenteil (5") des Oberteils (2) um zumindest ¾ des Umfangs (U'), vorzugweise des gesamten Umfangs (U') des Oberteils (2) und das Seitenteil (5') des Unterteils (3) passend zum Seitenteil (5") des Oberteils (2) um zumindest ¾ des Umfangs (U), vorzugweise des gesamten Umfangs (U) der Hose (3) erstreckt.

7. Kleidungsstück nach einem der Ansprüche 1 bis 6, **dadurch gekennzeichnet, dass** das Oberteil (2) an der Außenseite eine weitere Tasche (16, 16') zur Aufnahme und zum Transport einer medizinischen Pumpe aufweist, wobei die weitere Tasche (16, 16') eine Öffnung (26, 26') aufweist, welche von der Seite zugänglich ist und eine Größe aufweist, dass die Pumpe in die Öffnung (26, 26') steckbar ist, wobei die Öffnung (26, 26') der weiteren Tasche (16, 16') Mittels eines Verschlusses (18; 18') verschließbar ist, vorzugsweise mittels eine Reißverschlusses oder eines Klettverschlusses.

8. Kleidungsstück nach einem der Ansprüche 1 bis 7, **dadurch gekennzeichnet, dass** Tasche (6, 6', 16, 16`; 19) zur Aufnahme und zum Transport der medizinischen Pumpe integrierte Verstärkungsbänder (9) aufweist.

9. Kleidungsstück nach einem der Ansprüche 1 bis 8, **dadurch gekennzeichnet, dass** Tasche (6, 6`; 16, 16`; 19) zur Aufnahme und zum Transport der medizinischen Pumpe wasserfest ausgebildet sind, vorzugweise eine durch eine wasserdichte Beschichtung.

10. Kleidungsstück nach einem der Ansprüche 1 bis 9, **dadurch gekennzeichnet, dass** die Tasche (6, 6`; 16, 16`; 26) eine Polsterung aufweist, welche sich im getragenen Zustand des Kleidungsstücks (1) zwischen Körper und Pumpe befindet.
